# EUROPEAN PATENT SPECIFICATION

(11) **EP 1 125 546 B1**
(45) Date of publication and mention of the grant of the patent: **09.06.2010**
(21) Application number: 99940668.9
(22) Date of filing: 06.09.1999
(51) Int. Cl.: A61B 5/022, A61B 5/0225, G06F 17/00

(54) **HEMODYNAMOMETER AND ITS CUFF BAND**
HEMODYNAMOMETER UND SEINE MANSCHETTE
HEMODYNAMOMETRE ET SA MANCHETTE

(30) Priority: 04.09.1998 JP 25139498; 02.04.1999 JP 9571399
(43) Date of publication of application: 22.08.2001
(73) Proprietor: TERUMO KABUSHIKI KAISHA, Tokyo 151-0072 (JP); Tochikubo, Osamu, Yokohama-shi, Kanagawa 240-0024 (JP)
(72) Inventor: TOCHIKUBO, Osamu, Yokohama-shi, Kanagawa-ken 240-0024 (JP); HABU, Yoshiyuki, Terumo Kabushiki Kaisha, Ashigarakami-gun, Kanagawa-ken 259-0151 (JP); KURIO, Masaru, Terumo Kabushiki Kaisha, Ashigarakami-gun, Kanagawa-ken 259-0151 (JP); SOUMA, Takahiro, Terumo Kabushiki Kaisha, Fujinomiya-shi, Shizuoka-ken 418-0004 (JP)
(74) Representative: Casalonga, Axel
(86) International application number: PCT/JP1999/004810
(87) International publication number: WO 2000/013583

(56) References cited:
- DE-A- 4 410 297
- JP-A- 2 021 842
- JP-A- 5 269 089
- JP-A- 7 116 138
- US-A- 4 144 879
- PATENT ABSTRACTS OF JAPAN vol. 0180, no. 43 (C-1156), 24 January 1994 (1994-01-24) -& JP 05 269089 A (A & D CO LTD), 19 October 1993 (1993-10-19)

## Description

The present invention relates to a sphygmomanometer for measuring the blood pressure on the basis of changes in oscillations of the artery wall which are caused by arterial pulsation resulting from changes in the cuff pressure and, more particularly, to a sphygmomanometer and cuff characterized in a systolic blood pressure determination method.

An apparatus for measuring the systolic blood pressure of the person to be examined is used as follows. An arm band, i.e., a cuff is wound on the arm or wrist of the person to be examined, and this cuff is first pressurized to a pressure value well exceeding the systolic blood pressure to close the artery and stop the blood flow. Then, the cuff pressure is gradually reduced, and the sounds, (so-called Korotkoff sounds, K-sounds) generated when the stopped blood again starts flowing, are listened to. The cuff pressure at which the first sound is heard is regarded as the systolic blood pressure. This method is called auscultatory method. The method is simple and can detect the generation of blood flow relatively accurately, albeit indirectly. Hence, the method is in widespread use.

Unfortunately, this method requires a microphone to detect the Korotkoff sounds. Since this microphone readily picks up ambient noises and cuff scratch noises produced by body movement of the person to be examined, detection errors often occur.

An oscillometric method is known for use with an apparatus not using a microphone. In this method, a pulsewave generated from the artery is detected by continuously measuring the internal pressure of the cuff, and the systolic blood pressure is measured from the amplitude of this pulsewave.

Compared to the auscultatory method, the oscillometric method is not easily influenced by ambient noises and cuff cloth scratch noises, because no microphone is necessary. However, this oscillometric method cannot detect the moment of opening of the closed artery. Therefore, a statistical method as disclosed in Japanese Patent Publication No. 61-40416 is used. In this method, the maximum value of the obtained amplitudes is detected, and the cuff pressure when the amplitude value is 1/2 this maximum value is regarded as the systolic blood pressure.

The following methods are also used instead of the above oscillometric method. That is, a light-emitting/light-receiving element sensor which uses attenuation caused by the absorption and reflection of infrared light by the blood flow is placed on the skin of a peripheral portion of a human body, such as a finger, where the artery is present near the skin surface, and the generation of blood flow is detected by this sensor. Also, an ultrasonic method detects the generation of blood flow by oscillations of the blood vessel wall by ultrasonic waves, by using an ultrasonic sensor in place of the light-emitting/light-receiving element sensor. In each of these methods, the moment of resumption of the stopped blood flow is detected by the detected value of the sensor, and the cuff pressure at that moment is regarded as the systolic blood pressure.

Unfortunately, a statistical method such as the oscillometric method ignores differences between individuals. Therefore, errors can occur by variations caused by these individual differences, or by those variations in the measurement conditions which are caused by the generation of pulsewave, e.g., the cuff size, the cuff air volume which is affected by the way the cuff is wound, and the depressurization speed.

Also, a method of detecting the moment of resumption of the blood flow by an optical element sensor or an ultrasonic sensor is applicable only to blood vessels present near the skin surface. Hence, no satisfactory performance can be obtained from a portion, such as the upper arm, where a blood vessel runs at depth in a living body. Additionally, the sensor element must be accurately set close to a blood vessel near the skin surface. Therefore, if the cuff is attached in a wrong manner, accurate measurement cannot be performed. This sometimes makes accurate systolic blood pressure measurement impossible.

Japanese Patent Laid-Open No. 63-150051 disclosed a sphygmomanometer having a small cuff dedicated to pulsewave detection, placed on the peripheral side below a large cuff that is exclusively used to stop the blood flow. This small cuff detects a pulsewave, generated by the blood flow, which has a frequency component lower than those of the K-sounds, as a pressure change, instead of detecting the K-sounds having frequency components overlapping the frequency of external noises, such as cuff cloth scratch noises, which is a drawback of the auscultatory method.

The signal detected by this small cuff is similar to the signal detected by the oscillometric method. The signal can be detected even if an artery of interest does not run near the skin surface or even if the cuff is not accurately positioned on an artery of interest. Hence, the method using this small cuff is superior in operability to the method using the optical element sensor and the ultrasonic method.

This small cuff can detect the moment of resumption of the blood flow. However, since the small cuff is placed on the cuff peripheral side of the large cuff, the detected signal is small if the pulse is weak, so accurate measurement cannot be performed.

Also, to detect a signal only from this small cuff, a tubule for damping a signal detected by the large cuff is inserted between the large cuff and a sensor. If this tubule is too small, a pressure difference is produced between the sensor and the large cuff depending on the depressurization speed of the cuff. So, the inner diameter of the tubule can be decreased only to a certain extent. Consequently, the influence of the detected signal from the large cuff cannot be completely eliminated. This lowers the accuracy of detection of the resumption of blood flow.

Additionally, the detection of the diastolic blood pressure has the following drawback. That is, the frequency of the signal detected by the small cuff is lower than those of the K-sounds, so the K-sounds cannot be accurately detected. Accordingly, the Korotkoff's five-phase as the disappearing point of the K-sounds cannot be well detected. This makes it impossible to measure the diastolic blood pressure with probability equivalent to that of the auscultatory method.

JP 05-269089 discloses a hemomanometer comprising an outer pressure cuff- and-bag shaped as a belt and adapted to be wound around an upper arm to block an arterial bloodstream, an inner pressure cuff-and-bag almost at the center thereof to be wound around the arm, and a pressure sensor arranged in the inner pressure cuff-and-bag to detect changes in pulse wave to be propagated through leading liquid. A vibration shielding plate is interposed between the pressure sensor and the outer pressure cuff-and-bag to shield the propagation of vibrations as probable disturbance to the inner pressure cuff-and-bag through the outer pressure cuff-and-bag.

DE 44 10 297 A1 discloses an arrangement for blood pressure measurement comprising an inflatable cuff connected to a blood pressure measurement instrument working oscillatometrically. The cuff comprises a first measurement chamber and a second measurement chamber, each connected to respective first and second pressure sensors. The second, outer measurement chamber is arranged concentrically with, and thereby encloses, the first measurement chamber. The first and second measurement chambers are vibration- and pressure-insulated from each other by a substantially form-stable hard shell covered with a vibration-absorbing material on its surface facing the second measurement chamber.

### SUMMARY OF THE INVENTION

It is an object of the present invention to provide a sphygmomanometer having a systolic blood pressure determination method which is clear and accurate in principle, which is applicable to a portion, such as the upper arm, where a blood vessel runs depth in a living body, and which produces little variations by individual differences or measurement conditions.

It is another object of the present invention to provide a sphygmomanometer having high-sensitivity systolic blood pressure measurement capability and diastolic pressure detection capability by which the point of resumption of the blood flow can be detected with high sensitivity in a system which uses two cuffs, namely, large and small cuffs.

It is still another object of the present invention to provide an electronic sphygmomanometer and its cuff capable of preventing easy absorption of pulsewave oscillations (oscillations caused by arterial pulsation) by the use of a compression cuff and measurement cuff, and also capable of measuring an accurate blood pressure value by aligning a measurement portion with a blood stopped portion by placing the measurement cuff in a cuff central portion where the pressing force of the large compression cuff is large.

It is still another object of the present invention to provide a sphygmomanometer and its cuff capable of measuring an accurate blood pressure value by insulating oscillations caused by arterial pulsation, which, even though the blood flow in a central portion is stopped, are transmitted to a measurement cuff from the outside, particularly from a cuff upstream portion where the pressing force of a compression cuff is small.

To solve the above problems, the present invention includes the following arrangements.

As claimed, there is provided a sphygmomanometer for measuring a blood pressure on the basis of a change in oscillation of the artery wall caused by arterial pulsation when a cuff pressure changes comprising a cuff, said cuff comprising: a compression cuff adapted to press a blood vessel; and a measurement cuff which is adapted to measure the pulsation.

According to the invention, said measurement cuff is smaller than said compression cuff and placed in substantially the center of said compression cuff in a direction in which a blood vessel to be pressed by said compression cuff runs, and when blood pressure is to be measured, said measurement cuff is covered with said compression cuff so that said measurement cuff comes in close contact with a portion to be measured.

Further according to the invention, the sphygmomanometer further comprises oscillation insulating means adapted to prevent the transmission of oscillations from the upstream side of a blood vessel to said measurement cuff via said compression suff, said oscillation insulating means comprising a cushioning material placed on that contact surface between said compression cuff and a portion to be measured, which is in a blood vessel upstream portion of said measurement cuff.

Advantageously, said oscillation insulating means of the sphygmomanometer further comprise on the contact surface between said measurement cuff and said compression cuff, a cushioning material formed on the side of said compression cuff to absorb oscillations, and a rigid body formed on the side of said measurement cuff to insulate oscillations.

The sphygmomanometer may further comprise a connecting portion between the compression cuff and the measurement cuff, and wherein said oscillation insulating means further comprises a tubule provided in at least a part of the connecting portion. Said oscillation insulating means may further comprise an air chamber connected with the connecting portion between the compression cuff and the measurement cuff.

This sphygmomanometer further comprises a time measurement unit for measuring the time from the rise of the oscillation (wave form) to the peak detected by the peak detecting means, and a comparator for comparing the time measured by the time measurement unit with a predetermined time, wherein the plurality of pieces of peak information are selected in accordance with an output from the comparator.

The blood pressure determining means determines the systolic blood pressure on the basis of a value obtained by comparing the peak values (magnitudes) and/or the amplitude values (magnitudes) and/or the phase differences between a plurality of peaks detected by the peak detecting means.

As stated above in the sphygmomanometer of the present invention, the measurement cuff is placed in substantially the center of the compression cuff in a direction in which a blood vessel to be pressured by the compression cuff runs, and measures the oscillation, and the measurement cuff is smaller than the compression cuff and, when a blood pressure is to be measured, the measurement cuff is covered with the compression cuff so that the measurement cuff comes in close contact with a portion to be measured.

That is, in substantially the center of the inside of the compression cuff (to be also referred to as a large cuff) where the blood flow is stopped, the pulsewave measurement cuff (to be also referred to as a small cuff), which detects blood flow generated when the cuff pressure is gradually reduced from the blood stopped state as a pressure change, is placed. A pressure sensor for sensing the cuff pressure and a pulsewave is connected to the small cuff by an air tube. The large cuff is connected to this pressure sensor via an acoustic filter in order to attenuate pulsewaves detected by the large cuff. This acoustic filter comprises a tubule and air tank and passes only a DC component of the large.cuff pressure.

The cuff comprises first oscillation insulating means for preventing the transmission of oscillations from the compression cuff to the measurement cuff. This first oscillation insulating means comprises a first oscillation insulating mechanism formed on the contact surface between the measurement cuff and the compression cuff. The first oscillation insulating mechanism comprises an elastic material formed on the side of the compression cuff to absorb oscillations, and a rigid body formed on the side of the measurement cuff to insulate oscillations. When the measurement cuff and the compression cuff are connected, the first oscillation insulating means comprises a second oscillation insulating mechanism for insulating oscillations transmitted in a connecting portion between the measurement cuff and the compression cuff. This second oscillation insulating mechanism comprises a tubule formed in at least a part of the connecting portion between the measurement cuff and the compression cuff. The second oscillation insulating mechanism comprises an air chamber formed in the connection portion between the measurement cuff and the compression cuff to damp air oscillations.

The cuff further comprises third oscillation insulating means for preventing the transmission of oscillations from the upstream side of a blood vessel to the compression cuff. This third oscillation insulating means comprises a cushioning material placed on that contact surface between the compression cuff and a portion to be measured, which is in a blood vessel upstream portion of the measurement cuff.

That is, to attenuate pulsewaves detected by the large cuff and transmitted from the large cuff to the small cuff, a raised cushioning material is formed inside the large cuff, and a raised cushioning material and a highly rigid backing made of a rigid plate are stacked between the small cuff and the large cuff.

A portion of the large cuff located outside during blood pressure measurement is covered with a non-elastic material having a cut for reducing a dead volume is formed in the material.

To detect the generated blood flow and determine the systolic blood pressure, the sphygmomanometer comprises a peak detecting means formed inside the large cuff to detect peaks of a pulsewave component of a pressure signal detected by the small cuff, and determining means for detecting the generation of the blood flow and determining the systolic blood pressure by using a plurality of pieces of peak information per one pulsewave detected.

The sphygmomanometer may further comprise a time measurement unit for measuring the time from the rise of the pulsewave (wave form) to the generation of the peaks detected by the peak detecting means, and a comparator for comparing the time measured by the time measurement unit with a predetermined time, wherein the plurality of pieces of peak information are selected in accordance with an output from the comparator.

In this sphygmomanometer, the blood pressure determining means determines the systolic blood pressure on the basis of a value obtained by comparing the peak values (magnitudes) and/or the amplitude values (magnitudes) and/or the phase differences between a plurality of peaks detected by the peak detecting means.

With the above arrangement, the small cuff can be placed on the blood stopped point of the large cuff. In determining the systolic blood pressure, the generation point of a blood flow can be detected with high sensitivity. Also, not only the tubule but an air tank is connected between the large cuff and the pressure sensor. Accordingly, the passing frequency band of a low-pass filter composed of the tubule and the air tank can be set at a low frequency. This makes it possible to attenuate those pulsewaves detected by the large cuff, which prevent the detection of blood flow generation by the small cuff. Furthermore, the transmission of pulsewaves detected by the large cuff to the small cuff is minimized by the raised cushioning material positioned on that side of the cuff, which is close to the heart, and by the cushioning material formed by stacking the raised cushioning material and the highly rigid backing made of a rigid plate between the small cuff and the large cuff.

These components allow high-sensitivity detection of the resumption of a blood flow. In particular, it is possible to implement a sphygmomanometer improved in measurement ability when pulses are weak, such as when the patient has a serious illness.

In detecting the generation of a blood flow with this arrangement, to eliminate pulsation in the upper stream side of the cuff and accurately detect the blood flow generation point, the sphygmomanometer of the present invention detects at least two peaks generated when the blood flow passes through the cuff portion in synchronism with the heartbeat. The first peak is observed even when the cuff pressure is equal to or higher than the systolic blood pressure, i.e., even when the artery is closed. Therefore, this first peak presumably indicates the state in which the blood flow generated by the heartbeat enters the upstream side of the portion where the artery is closed. When the pressure is gradually reduced from the state in which the artery is closed, the blood vessel is opened, and the blood starts flowing toward peripheral portions. In this state, the measurement cuff detects the second peak which lags behind the first peak described above.

That is, since the measurement cuff is placed in substantially the center of the inside of the compression cuff, it is possible to discriminate between the first peak generated by the influence of the upstream side of a portion where the artery is closed under the cuff, and the second peak generated when the blood again starts flowing.

The sphygmomanometer of the present invention detects the above two peaks, detects the blood flow resumption point from changes in the peak values or phases of the peaks when the blood again starts flowing, and determines the cuff pressure at that resumption point as the systolic blood pressure. Therefore, the measurement principle is clearer and the reliability for measurement values is higher than the oscillometric method.

Non-limiting embodiments of the present invention will be explained in more detail with reference to the enclosed drawings, in which:
Fig. 1 is a schematic view showing the arrangement of a sphygmomanometer according to an embodiment of the present invention;
Fig. 2 is a view showing the arrangement of a cuff according to this embodiment of the present invention;
Fig. 3 is a longitudinal sectional view showing the state in which the cuff of this embodiment of the present invention is wound on the upper arm of an object to be examined;
Fig. 4 is a view showing examples of oscillation waveforms detected by a pressure sensor 16;
Fig. 5 is a block diagram of a section for determining the systolic blood pressure in this embodiment;
Fig. 6 is a flow chart for explaining the operation of the sphygmomanometer of this embodiment;
Fig. 7 is a graph showing a change in the pressure of a measurement cuff 12 of this embodiment;
Fig. 8 is a view showing a change in the pressure of a measurement cuff 12 of a second embodiment;
Fig. 9 is a view showing the arrangement of a cuff of this embodiment;
Fig. 10 is a longitudinal sectional view (a section in a direction in which the upper arm extends) viewed in a direction A of Fig. 1, when the cuff of this embodiment is wound on the upper arm;
Fig. 11 is a cross-sectional view (a section of the upper arm) viewed in a direction B of Fig. 1, when the cuff of this embodiment is wound on the upper arm;
Fig. 12 is a view showing the results of measurements of an oscillation wave pulse by the measurement cuff and the compression cuff of this embodiment; and
Figs. 13A and 13B are graphs showing the comparison between a pulsewave measured by the measurement cuff of this embodiment and a conventional measurement result.

To further clarify the features of a sphygmomanometer based on the present invention, embodiments thereof are described below with reference to the accompanying drawings.

### <Arrangement of sphygmomanometer of This Embodiment>

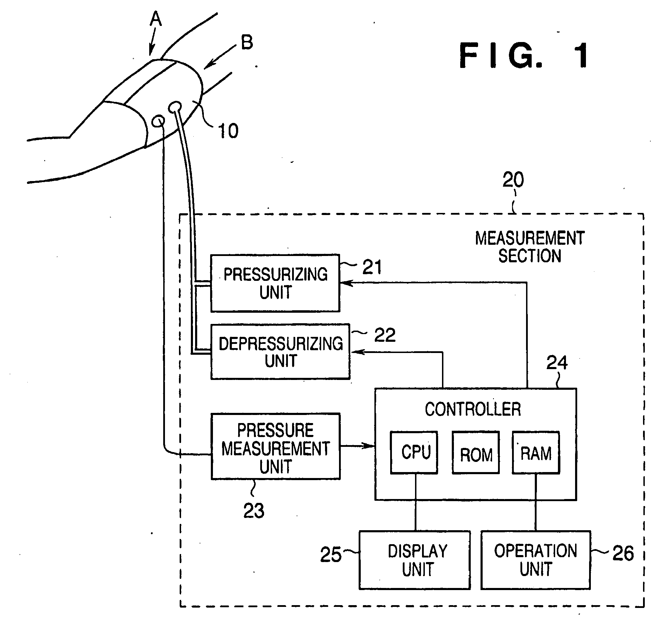

Fig. 1 is a schematic view showing the arrangement of a sphygmomanometer of this embodiment.

In Fig. 1, preference numeral 10 denotes a cuff having the arrangement of this embodiment; and 20, a measurement section which detects a blood pressure value from oscillations in the cuff internal pressure while controlling the pressure of the cuff 10. Details of the arrangement of the cuff 10 will be described later, so the arrangement of the measurement section 20 is described here.

Reference numeral 21 denotes a pressurizing unit; and 22, a depressurizing unit. In a sphygmomanometer which performs measurement only during depressurization, the pressurizing unit 21 rapidly pressurizes to a pressure exceeding the systolic blood pressure value and, after this pressurization is stopped, the depressurizing unit 22 depressurizes at a constant rate of, e.g., 2 to 3 mmHg/sec, under the control of a controller 24. A pressure measurement unit 23 receives an electrical signal from a pressure sensor (inside the cuff, not shown) of this embodiment, and outputs a digital pressure value to the controller 24. This pressure measurement unit 23 can also include circuits such as a low-pass filter or a peak holding circuit and output the amplitude value of a oscillation waveform.

The controller 24 comprises a CPU for controlling arithmetic operations, a ROM storing control programs and fixed parameters, and a working RAM for temporary storage. This controller 24 stores the output value from the pressure measurement unit 23 into the RAM in one-to-one correspondence with the current cuff pressure. The controller 24 determines the blood pressure value from the pattern of a change in this output value and displays the blood pressure value on a display unit 25. An operation unit 26 includes operation buttons such as a reset button and start button.

### <Arrangement of Cuff of This Embodiment>

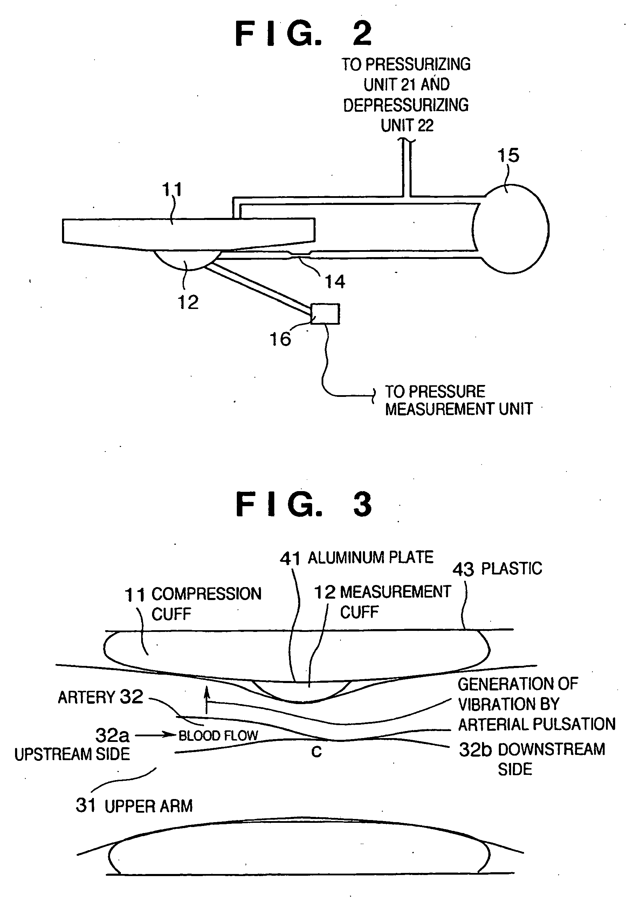

Fig. 2 is a view showing the arrangement of the cuff 10 of this embodiment.

In Fig. 2, reference numeral 11 denotes a compression cuff for pressing a blood vessel. This compression cuff 11 must be of sufficient size to apply a sufficient blood stopping pressure to a portion to be measured (this compression cuff will also be called a compression large cuff). A measurement cuff 12 is formed in a substantially central portion of the compression cuff 11 to detect pressure pulsewave oscillations. This measurement cuff 12 must be as small as possible to decrease the attenuation of wave height caused by air in the cuff (this measurement cuff will also be called a measurement small cuff). In the central portion of the compression cuff 11 where the measurement cuff 12 is formed, a blood vessel is closed by pressure, and this closing pressure is a maximum. A fluid resistor (tubule) 14 and an air chamber (air tank) 15 function as an acoustic filter for reducing or insulating oscillation noise applied to the measurement cuff 12. In this embodiment, a 26-gage cannula is used as this fluid resistor 14, and the fluid resistor 14 is preferably positioned as close as possible to the measurement cuff 12. The air chamber 15 functions as a compliance component of the acoustic filter. A pressure sensor 16 detects the internal pressure of the measurement cuff 12. The measurement cuff 12 and this pressure sensor 16 are desirably connected by a tube having large rigidity. The sensor can also be placed inside the measurement cuff 12.

Fig. 3 is a longitudinal sectional view (a section in a direction in which the upper arm extends) viewed in a direction A of Fig. 1, when the cuff 10 of this embodiment is wound on an upper arm 31. Referring to Fig. 3, the compression cuff 11 is pressurized to close a blood vessel 32 at a point C, thereby stopping the blood flow from an upstream side 32a to a downstream side 32b. Reference numeral 43 denotes a backing made of a non-elastic rigid plate (e.g., plastic) for covering and fixing the outside of the compression cuff 11, in order to allow the pressure of the compression cuff 11 to efficiently function as a blood stopping pressure. To improve the adhesion between the upper arm and the backing 43 and thereby reduce a dead volume, a cut (not shown) is desirably formed in the backing 43 in a direction substantially perpendicular to the longitudinal direction of the cuff 10.

As shown in Fig. 3, when the blood vessel at the point C is closed, no oscillations by arterial pulsation are transmitted to the downstream side of this point C because the blood flow is stopped. However, oscillations by arterial pulsation are transmitted to the upstream side of the point C and detected by the pressure sensor 16.

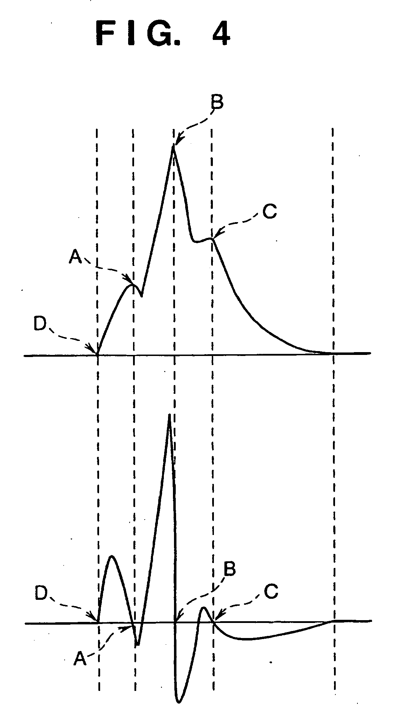

When the compression cuff 11 is gradually depressurized from the blood stopped state shown in Fig. 3, the blood vessel at the point C is opened, and the blood starts flowing in a direction toward peripheral portions. The upper waveform in Fig. 4 indicates an oscillation waveform detected by the pressure sensor 16 when the blood starts flowing. First and second peaks A and 8 of this upper waveform in Fig. 4, detected by the measurement cuff 12, are generated by a change in the blood flow which passes through the cuff portion in synchronism with the heartbeat. In the sphygmomanometer having the measurement cuff inside the compression cuff as in the present invention, two peaks appear when the blood flow passes through the cuff portion in synchronism with the heartbeat. A third peak C is generated by reflection of the pressure pulsewave arriving at a peripheral portion. Since this third peak C is not used in the present invention, the peak can also be processed by, e.g., filtering so as not to be detected. Reference symbol D denotes the start point of the rise of the pulsation.

The lower waveform in Fig. 4 is obtained by differentiating the upper oscillation waveform in Fig. 4, in order to detect the peak points.

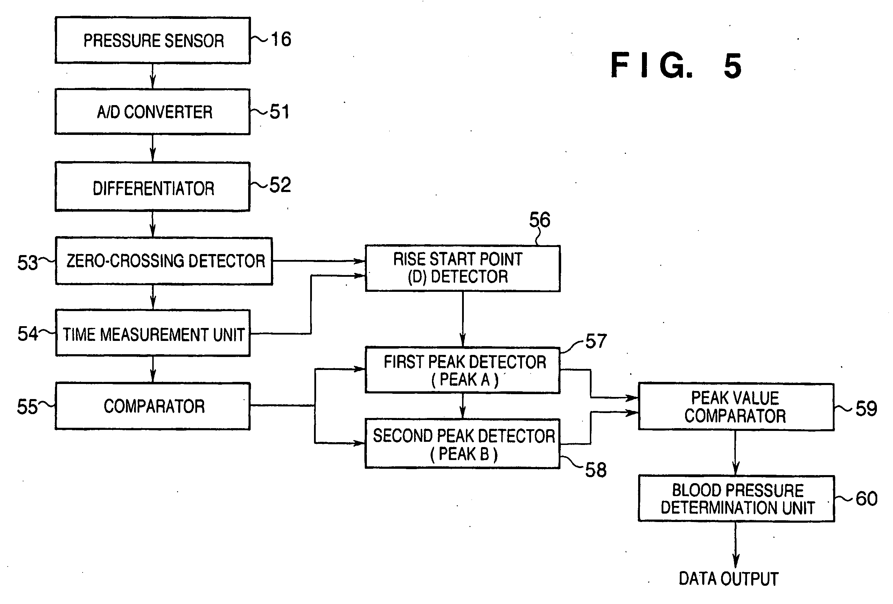

Fig. 5 is a block diagram showing a process for determining the systolic blood pressure in this embodiment. Referring to Fig. 5, an output signal from the pressure sensor 16 is subjected to amplification and noise discrimination by, e.g., a filter and amplifier, and A/D-converted by an A/D converter 51. After that, the signal is output as a digital signal to a differentiator 52. This differentiator 52 differentiates the digital signal and outputs the differentiated signal to a zero-crossing detector 53. The zero-crossing detector 53 detects a point at which the output value from the differentiator is zero, and outputs the detected point to a rise start point detector 56. The rise start point detector 56 detects the rise start point D shown in Fig. 4, from the signal obtained by the differentiator 52. The zero-crossing detector 53 sends the signal to a time measurement unit 54 whenever detecting the point (zero-crossing point) at which the value is zero. The time measurement unit 54 measures the time from the rise start point D to this zero-crossing point, and sends a signal to a comparator 55. The comparator 55 compares a prestored first predetermined time (range) with the time measured by the time measurement unit 54. If the relationship between the measured time and the predetermined time falls within an allowable range, the comparator 55 sends a signal to a first peak detector 57. The first peak detector 57 receives this signal and detects it as the first peak A shown in Fig. 4. If the comparison result obtained by the comparator 55 falls outside the predetermined time range, no detection is performed, and the next pulsewave is detected. When a zero-crossing point at which the value changes from positive to negative is detected after the first peak A is detected, the comparator 55 compares the time from the first peak A to this zero-crossing point with a prestored second predetermined time (range). If the measured time falls within the predetermined time range, the comparator 55 sends a signal to a second peak detector 58. The second peak detector 58 receives this signal and detects the second peak B shown in Fig. 4. If the comparison result obtained by the comparator 55 falls outside the predetermined time range, no detection is performed, and the next pulsewave is detected.

When the two peaks A and B are detected, the systolic blood pressure is determined by a peak value (magnitude) comparator 59 which compares the magnitude of the two peaks, and by a blood pressure determination unit 60 which determines the systolic blood pressure on the basis of the data from the peak value comparator 59.

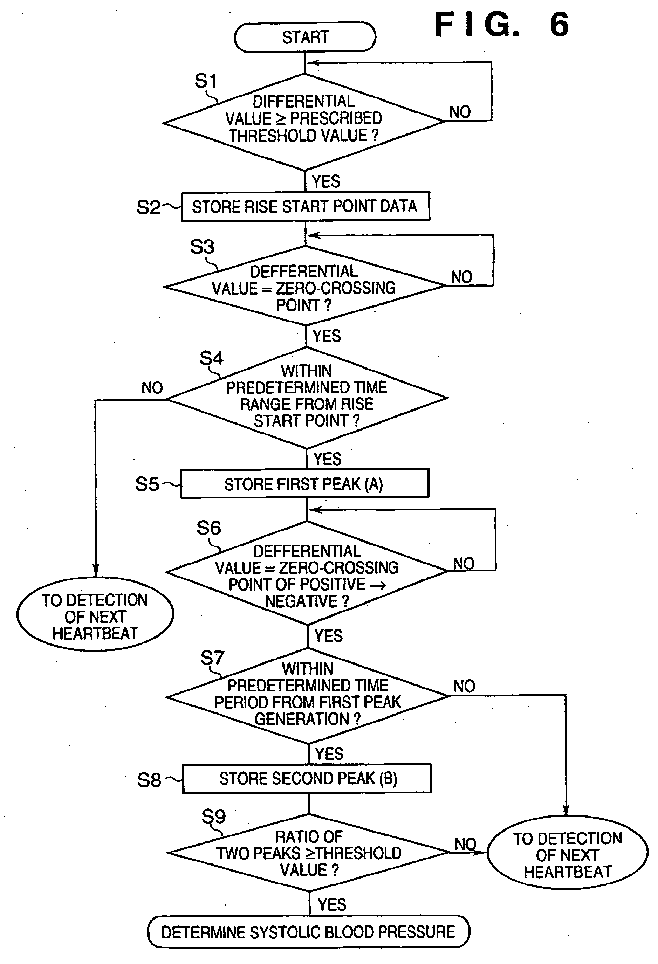

Fig. 6 explains the operation of each unit in Fig. 5 by a flow chart. In step 51, whether the differential value calculated by the differentiator 52 is equal to or larger than a threshold value is checked. If the differential value is equal to or larger than the threshold value, the flow advances to step S2, and the data is stored by regarding this point as the rise start point D. In step S3, a point, at which the differential value is 0 after the rise start point is detected, is searched. If this point is found, the flow advances to step S4, and whether the point appears within a predetermined time range from the rise start point is checked. If the point appears within the predetermined time range, this peak is regarded as the first peak A shown in Fig. 4 and stored in step S5, and the height (the peak value) of the pulsewave at this first peak A is calculated from the original waveform. In step S6, a zero-crossing point at which the differential value changes from positive to negative after the first peak A is detected is searched for. In step S8, this point is stored as the second peak B, and the height (the peak value) of the pulsewave at that point is calculated from the original waveform. If no peak is detected during the predetermined time after the first peak A is detected, it is determined in step S7 that the waveform does not have the second peak B, so the flow shifts to detection of the oscillation waveform of the next pulse. If both the first and second peaks are detected, the ratio of, the peak values of these two peaks are compared in step S9. If the ratio is equal to or larger than a threshold value, the cuff pressure at this time is saved as systolic blood pressure. If the ratio is smaller than the threshold value, it is determined that the cuff pressure is higher than the systolic blood pressure. Accordingly, the waveform of the next pulse is similarly processed in succession.

If it is determined in step S4 that the peak appears outside the predetermined time range from the rise start point, the flow shifts to detection of the next heartbeat.

### <First Embodiment>

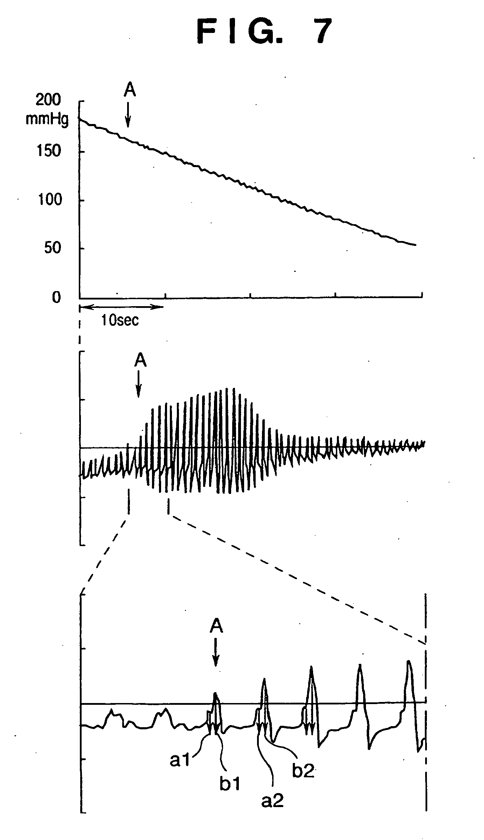

The upper waveform shown in Fig. 7 indicates a change in the pressure of the measurement cuff 12 when the compression cuff 11 and the measurement cuff 12 are pressurized to the systolic blood pressure or higher and then gradually depressurized. The middle waveform in Fig. 7 is obtained through a filter having a time constant of 0.1 sec, in order to extract an AC component of the pressure change of the upper waveform in Fig. 7. The lower waveform in Fig. 7 is obtained by enlarging the waveform near a point A of the middle waveform in Fig. 7 in the direction of the abscissa (time axis).

In the lower waveform in Fig. 7, two peaks appear in the rising portion of a oscillation waveform of each pulse. The first peak is the peak A in Fig. 4, which is transmitted via the compression cuff 11. The second peak is the peak 8 in Fig. 4. Let aₙ (n = 1, 2, 3, ...) be the height from the rise start point of the peak A and bₙ (n = 1, 2, 3, ...) be the height of the peak B, the height aₙ of the peak A changes little around the point A. On the other hand, the height bₙ of the peak B becomes larger than that of the peak A after the point A, indicating that the amplitude of bₙ increases as depressurization progresses. This shows that the stopped blood flow again starts flowing at the point A, so the cuff pressure at that point can be regarded as the systolic blood pressure. Accordingly, the systolic blood pressure can be detected by setting a threshold value for a ratio bₙ/aₙ of the height of the peak B to the height of the peak A and detecting a point at which this ratio exceeds the threshold value.

In this embodiment, oscillations of the artery wall are detected by using an AC component of the change in the internal pressure of the measurement cuff 12. To detect an oscillation, however, it is also possible to use a method of detecting a change in the arm circumference of a blood stopped portion by using a strain gage, or a method of detecting a change in the volume of a blood stopped portion by using an impedance method.

### <Second Embodiment>

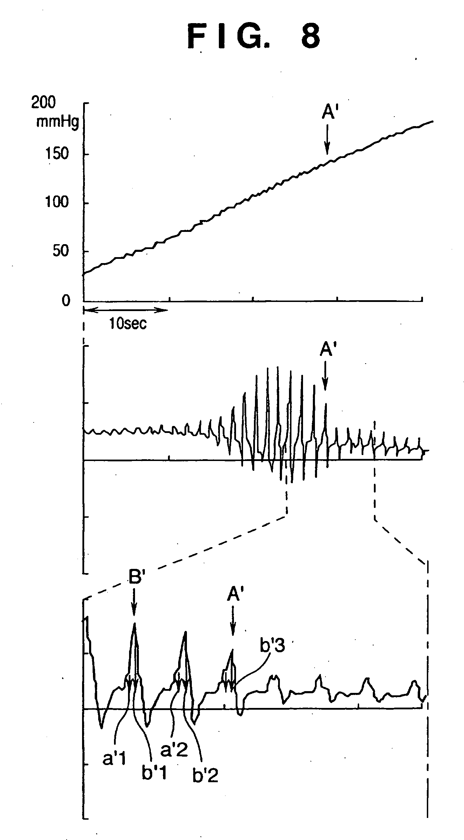

In the above first embodiment, the pressure of the measurement cuff 12 is measured while the measurement cuff 12 is gradually depressurized after being pressurized so that the artery is closed. In this second embodiment, however, the systolic blood pressure is calculated by measuring the pressure of the measurement cuff 12 during pressurization. The upper waveform in Fig. 8 shows a change in the pressure of the measurement cuff 12 when the cuffs 11 and 12 are gradually pressurized. The middle waveform in Fig. 8 is obtained through a filter having a time constant of 0.1 sec, in order to extract an AC component of the pressure change of the upper waveform in Fig. 8. The lower waveform in Fig. 8 is obtained by enlarging the waveform near a point A' of the middle waveform in Fig. 8 in the direction of the abscissa (time axis).

In the lower waveform in Fig. 8, two peaks appear in the rising portion of a oscillation waveform of each pulse. The first peak is the peak A in Fig. 4, and the second peak is the peak B in Fig. 4. Letting a'ₙ (n = 1, 2, 3, ...) be the height from the rise start point of the peak A and b'ₙ (n = 1, 2, 3, ...) be the height of the peak B, the height a'ₙ of the peak A changes little around the point A'. On the other hand, the height b'ₙ of the peak B abruptly decreases after the point A', indicating that the amplitude decreases as depressurization progresses. This shows that the artery is closed and the blood flow is stopped at the point A', so the cuff pressure at that point can be regarded as the systolic blood pressure. Accordingly, the systolic blood pressure, can be detected by setting a threshold value for a ratio b'ₙ/a'ₙ of the height of the peak B to the height of the peak A and detecting a point at which this ratio becomes smaller than the threshold value.

In this embodiment, the peak value is calculated as a height to the peak by regarding a reference point of the peak value as the rise start point. However, any arbitrary peak value calculation method can be used as long as the method is not contradictory to the features of the present invention. The peak detection method can also be any arbitrary method. Furthermore, although the ratio of the wave height of the second peak to the wave height of the first peak is used in this embodiment, some other comparison methods (e.g., a method using a phase difference) are also usable.

As described above, this embodiment uses a plurality of peaks generated when the blood flow passes through the cuff portion in synchronism with contraction of the heart. Accordingly, it is possible to provide a sphygmomanometer having a systolic blood pressure determination method which is clear and accurate in principle and which reduces the burden on a patient.

### <Arrangement of Improved Cuff of This Embodiment>

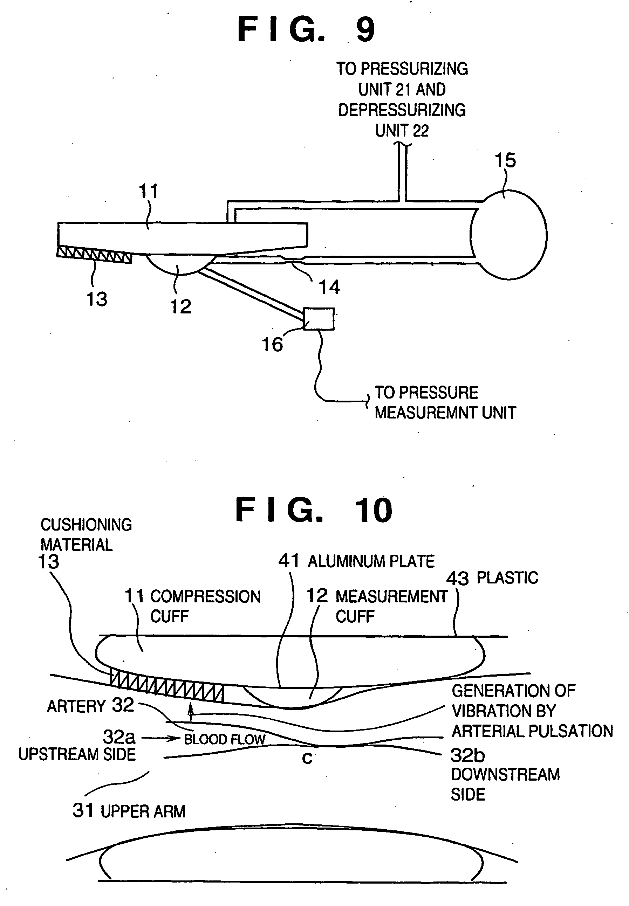

Fig. 9 is a view showing the arrangement of an improved cuff 10 of this embodiment. The same reference numerals as in Figs. 2 and 3 denote components having the same functions in Fig. 9.

Referring to Fig. 9, a compression cuff 11 for pressing the artery must be of a size large enough to apply a sufficient blood stopping pressure to a portion to be measured. A measurement cuff 12 for detecting oscillation pulsewaves must be as small as possible to decrease the absorption of oscillation pulsewaves. A cushioning material 13 prevents the transmission of oscillations from the upstream side of a blood vessel to the compression cuff as will be described later. A tubule 14 functions as an acoustic filter for shielding the measurement cuff 12 from oscillations of the compression cuff 11, a pressurizing unit 21, and a depressurizing unit 22. In this embodiment, a 26-gage cannula is used. This filter 14 is preferably positioned as close as possible to the measurement cuff 12. Reference numeral 15 denotes a cuff for children, as an example of an air chamber which functions as a damper for absorbing oscillations of the compression cuff 11, the pressurizing unit 21, and the depressurizing unit 22. A pressure sensor 16 detects the internal pressure of the measurement cuff 12. The measurement cuff 12 and this pressure sensor 16 are desirably connected by a non-elastic tube. The sensor can also be placed inside the cuff.

Fig. 10 is a longitudinal sectional view (a section in a direction in which the upper arm extends) viewed in the direction A of Fig. 1, when the cuff 10 of this embodiment is wound on an upper arm 31. Referring to Fig. 10, the compression cuff 11 is pressurized to close a blood vessel 32 at a point C, thereby stopping the blood flow from an upstream side 32a to a downstream side 32b.

As shown in Fig. 10, when the blood vessel at the point C is closed, no oscillations by arterial pulsation are transmitted to the downstream side of this point C because the blood flow is stopped. However, oscillations by arterial pulsation cannot be suppressed on the upstream side of the point C. To prevent these oscillations on the upstream side from being picked up by the compression cuff 11 and transmitted to the measurement cuff 12, it is necessary to provide means for preventing the transmission of the oscillations by arterial pulsation on the upstream side to the measurement cuff 12, such as the cushioning material 13, the air chamber 15, the tubule 14, and an aluminum plate 41, as shown in Fig. 9.

Each component for preventing the transmission of the oscillations by arterial pulsation on the upstream side to the measurement cuff 12 will be described in detail below.

Reference numeral 13 denotes a cushioning material, which is also shown in Fig. 9, for preventing the transmission of the oscillations from the upstream side of the artery to the compression cuff. "Velcro" or the like is suitable as the material. Reference numeral 41 denotes an oscillation shielding member for shielding the measurement cuff 12 from oscillations of the compression cuff 11, the pressurizing unit 21, and the depressurizing unit 22, in cooperation with the tubule 14 and the air chamber 15. This oscillation shielding member 41 includes a rigid body or a rigid plate such as an aluminum plate. Reference numeral 43 denotes a non-elastic rigid plate (e.g., plastic) for covering and fixing the outside of the compression cuff 11, in order to allow the pressure of the compression cuff 11 to efficiently function as a blood stopping pressure. This outer plastic fits on the arm regardless of the arm shape because the plastic has a certain degree of freedom in the circumferential direction of the arm but has a limited degree of freedom in the longitudinal direction of the arm. To reduce a dead volume between the arm and the plastic, a cut (not shown) is desirably formed in the plastic in a direction substantially perpendicular to the longitudinal direction of the cuff 10.

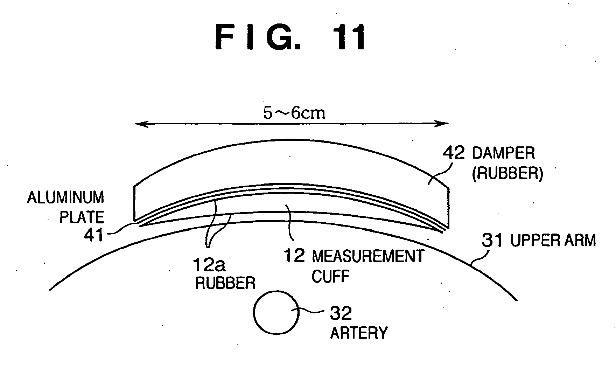

Fig. 11 is a cross-sectional view (a section of the upper arm) viewed in the direction B of Fig. 1, when the cuff 10 of this embodiment is wound on the upper arm 31. The compression cuff 11 is not shown in Fig. 11. Physical insulation between the measurement cuff 12 and the compression cuff 11 will be described in more detail below with reference to Fig. 11.

Rubber 12a forms the measurement cuff 12. An aluminum plate which is a rigid body 41 and insulates oscillations is formed along the outside of this rubber 12a. A damper 42 is formed outside this aluminum plate so as to cover the measurement cuff 12. An aluminum plate is preferably used as the rigid body 41, and a rubber plate is preferably used as the damper 42.

In this embodiment as described above, the measurement cuff is formed in a portion where the blood flow is stopped by the compression cuff, so accurate oscillation patterns can be obtained. In addition, the transmission of oscillations on the upstream side of the cuff to the compression cuff is prevented, and noise from the compression cuff, the pressurizing unit 21, and the depressurizing unit 22 to the measurement cuff is insulated. Therefore, large pulsewave oscillations of the artery in the cuff central portion can be detected.

The use of this measurement value allows accurate blood pressure value measurement.

That is, blood pressure values can be determined by a simple algorithm (e.g., a pressure higher than the systolic blood pressure is applied and, while this pressure is lowered, the blood pressure when the amplitude abruptly increases is detected as the systolic blood pressure, and the blood pressure when the amplitude abruptly decreases is detected as the diastolic blood pressure).

It is also possible to measure the amplitudes of both the compression cuff and the measurement cuff and detect the blood pressure when (the amplitude of measurement cuff/the amplitude of the compression cuff) abruptly increases as the systolic blood pressure and the blood pressure when this ratio abruptly decreases as the diastolic blood pressure.

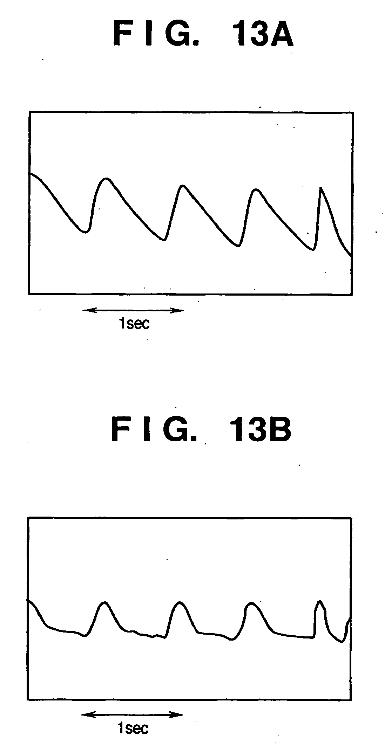

Additionally, this sphygmomanometer can also be used as a plethysmograph because the frequency characteristics are excellent as shown in Fig. 13A to be described later. It is also possible to use this sphygmomanometer to analyze the properties of a blood vessel by using the waveform of a pulsewave and as a continuous blood pressure monitor.

In this embodiment, compared to the conventional methods, only pulsation in the cuff central portion is detected, and high frequency components of pulsewaves can be accurately detected. This facilitates determination of the start point (systolic blood pressure) of pulsation or the point (diastolic blood pressure) at which pulsation abruptly decreases. This can increase the detection accuracy.

Also, the use of the small cuff permits detection of high frequency components of pulsewaves, compared to the conventional methods. This indicates that the properties, e.g., the hardness of a blood vessel can be determined by simultaneously measuring the blood pressure and the pulsewaveform.

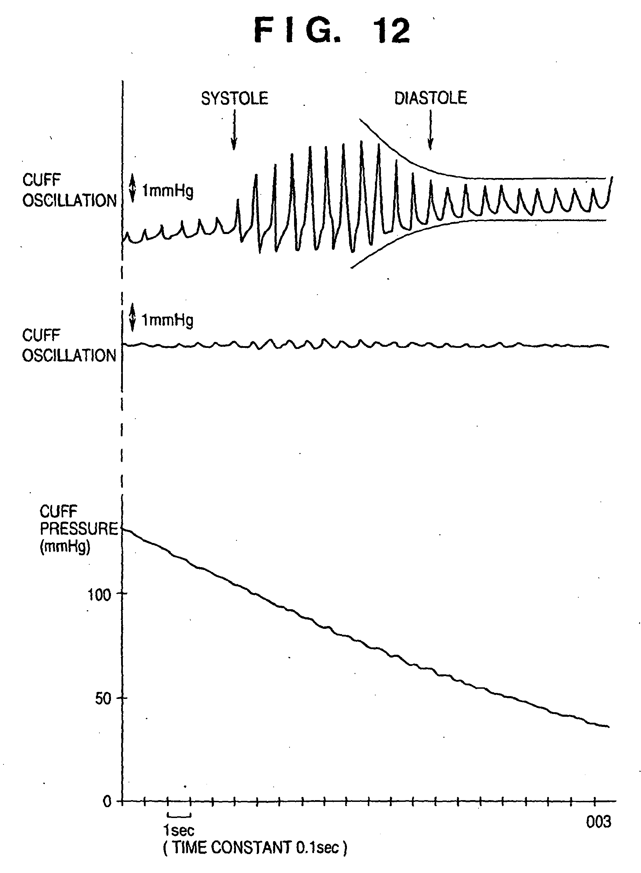

As described above, the cuff and the sphygmomanometer manufactured by the above arrangements have the characteristics: (1) oscillations by arterial pulsation on the upstream side of the cuff are insulated; and (2) the small measurement cuff improves the frequency characteristics of pulse waveforms. To clarify these characteristics, Figs. 12, 13A, and 13B show the results of measurements of pulsewave oscillations performed using this cuff.

The upper waveform in Fig. 12 is detected from the measurement cuff, and the middle waveform in Fig. 12 is detected from the compression cuff. Each waveform is obtained by extracting an AC component of a pressure change. The lower waveform in Fig. 12 indicates a change in the pressure of the measurement cuff.

As shown in Fig. 12, a pulsewave oscillation having neither distortion nor decay in the waveform and having a large amplitude can be obtained from the measurement cuff. This facilitates determination of the systolic blood pressure and the diastolic blood pressure, and makes measurements of accurate blood pressure values feasible.

Figs. 13A and 13B are graphs showing the pulsewave measurement results when the cuff pressure near the diastolic blood pressure value is kept constant. Fig. 13A shows a waveform detected from the measurement cuff of this embodiment. Fig. 13B shows a waveform detected by one large cuff of a conventional sphygmomanometer.

As shown in Figs. 13A and 13B, the pulsewave detected by the measurement cuff of this embodiment has neither distortion nor decay in its waveform and has a large amplitude, indicating good frequency characteristics. Hence, this sphygmomanometer can be used as a plethysmograph, used to analyze the properties of a blood vessel by using the waveform of a pulsewave, or used as a continuous blood pressure monitor.

With this structure of the cuff of this embodiment, it is possible to provide a sphygmomanometer and its cuff capable of preventing easy diffusion of pulsewave oscillations by the use of a compression cuff and measurement cuff, and also capable of measuring an accurate blood pressure value by aligning a measurement portion with a blood stopped portion by placing the measurement cuff in a cuff central portion where the pressing force of the large compression cuff is large.

Furthermore, it is possible to provide a sphygmomanometer and its cuff capable of measuring an accurate blood pressure value by insulating oscillations caused by arterial pulsation, which, even though the blood flow in a central portion is stopped, are transmitted to a measurement cuff from the outside, particularly from a cuff upstream portion where the pressing force of a compression cuff is small.

Preferred embodiments of the present invention have been described above. However, the present invention is not limited to the above embodiments, and various changes, additions, and modifications can be made within the scope described in the appended claims.

## Claims

1. A sphygmomanometer for measuring a blood pressure on the basis of a change in oscillation of the artery wall caused by arterial pulsation when a cuff pressure changes, comprising a cuff (10),
said cuff (10) comprising :
a compression cuff (11) adapted to press a blood vessel; and
a measurement cuff (12) which is adapted to measure the pulsation,
wherein said measurement cuff (12) is smaller than said compression cuff (1 1) and placed in substantially the center of said compression cuff (11) in a direction in which a blood vessel (32) to be pressed by said compression cuff (11) runs, and when blood pressure is to be measured, said measurement cuff (12) is covered with said compression cuff (11) so that said measurement cuff (12) comes in close contact with a portion to be measured, and wherein
the sphygmomanometer further comprises oscillation insulating means (13, 41, 42, 14, 15) adapted to prevent the transmission of oscillations from the upstream side (32a) of a blood vessel (32) to said measurement cuff (12) via said compression cuff (11), **characterized in that**
said oscillation insulating means comprise a cushioning material (13) placed on that contact surface between said compression cuff (11) and a portion to be measured, which is in a blood vessel upstream portion of said measurement cuff (12).

2. The sphygmomanometer according to claim 1 , wherein said oscillation insulating means further comprises on the contact surface between said measurement cuff (12) and said compression cuff (11), a cushioning material (42) formed on the side of said compression cuff to absorb oscillations, and a rigid body (41) formed on the side of said measurement cuff to insulate oscillations.

3. The sphygmomanometer according to claims 1 or 2, further comprising a connecting portion between the compression cuff (11) and the measurement cuff (12), and wherein said oscillation insulating means further comprises a tubule (14) provided in at least a part of the connecting portion.

4. The sphygmomanometer according to claim 3, wherein said oscillation insulating means further comprise an air chamber (15) connected with the connecting portion between the compression cuff (11) and the measurement cuff (12).

## Patentansprüche

1. Ein Blutdruckmessgerät zum Messen eines Blutdrucks auf der Grundlage einer Veränderung der durch arterielles Pulsieren verursachten Schwingung der Arterienwand, wenn sich ein Manschettendruck ändert, mit einer Manschette (10),
wobei die Manschette (10)
eine zum Drücken eines Blutgefässes ausgebildete Kompressionsdruckmanschette (11) und
eine zum Messen des Pulsierens ausgebildete Messmanschette (12) umfasst, wobei
die Messmanschette (12) in einer Richtung, in der ein durch die Kompressionsdruckmanschette (11) zu drückendes Blutgefäß (32) verläuft, kleiner als die Kompressionsdruckmanschette (11) und im wesentlichen in der Mitte der Kompressionsdruckmanschette (11) angeordnet ist, wobei wenn der Blutdruck gemessen werden soll, die Messmanschette (12) von der Kompressionsdruckmanschette (11) überdeckt ist, so dass die Messmanschette (12) mit einem zu messenden Bereich in engen Kontakt kommt, und wobei
das Blutdruckmessgerät ferner Schwingungsabschirmungsmittel (13, 41, 42, 14, 15) umfasst, die dazu ausgebildet sind, die Übertragung von Schwingungen von der stromaufwärtigen Seite (32a) des Blutgefässes (32) durch die Kompressionsdruckmanschette (11) zu der Messmanschette (12) zu verhindern, **dadurch gekennzeichnet, dass** die Schwingungsabschirmungsmittel ein Polstermittel (13) umfassen, das angeordnet ist auf der Kontaktoberfläche zwischen der Kompressionsdruckmanschette (11) und dem zu messenden Bereich, der in einem stromaufwärtigen Bereich des Blutgefässes in Bezug auf die Messmanschette (12) ist.

## Revendications

1. Sphygmomanomètre pour mesurer une tension artérielle d'après un changement d'oscillation de la paroi artérielle provoqué par le pouls artériel quand une pression de brassard est modifiée, comprenant un brassard (10),
ledit brassard (10) comprenant :
un brassard de compression (11) adapté pour comprimer un vaisseau sanguin ; et
un brassard de mesure (12) qui est adapté pour mesurer le pouls, dans lequel
ledit brassard de mesure (12) est plus petit que ledit brassard de compression (11) et est placé sensiblement au centre dudit brassard de compression (11) dans une direction dans laquelle est orienté un vaisseau sanguin (32) devant être comprimé par ledit brassard de compression (11), et quand la tension artérielle doit être mesurée, on recouvre ledit brassard de mesure (12) dudit brassard de compression (11) de sorte que ledit brassard de mesure (12) vient en contact étroit avec une partie à mesurer, et dans lequel
le sphygmomanomètre comprend en outre un moyen isolant des oscillations (13, 41, 42, 14, 15) adapté pour empêcher la transmission d'oscillations depuis le côté amont (32a) d'un vaisseau sanguin (32) vers ledit brassard de mesure (12) par l'intermédiaire dudit brassard de compression (11), **caractérisé en ce que** ledit moyen isolant des oscillations comprend un matériau d'amortissement (13) placé sur la surface de contact entre ledit brassard de compression (11) et une partie à mesurer, qui est dans une partie amont de vaisseau sanguin dudit brassard de mesure (12).
